# EUROPEAN PATENT SPECIFICATION

(11) **EP 2 536 727 B1**
(45) Date of publication and mention of the grant of the patent: **02.07.2014**
(21) Application number: 11704225.9
(22) Date of filing: 17.02.2011
(51) Int. Cl.: C07D 487/04, C07B 59/00, A61K 31/519, A61P 35/00

(54) **DEUTERATED PYRROLOPYRIMIDINE COMPOUNDS AS INHIBITORS OF CDK4/6**
DEUTERIERTE PYRROLOPYRIMIDIN VERBINDUNGEN ALS CDK4/6 INHIBITOREN
COMPOSÉS DEUTÉRIÉS DE LA PYRROLOPYRIMIDINE UTILISÉS EN TANT QU'INHIBITEURS DES CDK4/6

(30) Priority: 19.02.2010 US 306248 P
(43) Date of publication of application: 26.12.2012
(73) Proprietor: Novartis AG, 4056 Basel (CH)
(72) Inventor: BRAIN, Christopher Thomas, Cambridge Massachusetts 02139 (US); PEREZ, Lawrence Blas, Cambridge Massachusetts 02139 (US)
(74) Representative: Dyer, James
(86) International application number: PCT/EP2011/052365
(87) International publication number: WO 2011/101417

(56) References cited:
- WO-A1-2010/020675
- WO-A2-2007/140222
- US-A1- 2009 203 688
- MORIARTY K J ET AL: "The synthesis and SAR of 2-amino-pyrrolo[2,3-d]pyrimidines: A new class of Aurora-A kinase inhibitors", BIOORGANIC & MEDICINAL CHEMISTRY LETTERS, PERGAMON, ELSEVIER SCIENCE, GB, vol. 16, no. 22, 15 November 2006 (2006-11-15), pages 5778-5783, XP025106713, ISSN: 0960-894X, DOI: DOI:10.1016/J.BMCL.2006.08.080 [retrieved on 2006-11-15]
- NUTLEY BERNARD P ET AL: "Metabolism and pharmacokinetics of the cyclin-dependent kinase inhibitor R-roscovitine in the mouse.", January 2005 (2005-01), MOLECULAR CANCER THERAPEUTICS JAN 2005 LNKD- PUBMED:15657360, VOL. 4, NR. 1, PAGE(S) 125 - 139, XP002647196, ISSN: 1535-7163 abstract; figure 1
- FOSTER A B: "Deuterium isotope effects in the metabolism of drugs and xenobiotics: implications for drug design", ADVANCES IN DRUG RESEARCH, ACADEMIC PRESS, LONDON, GB, vol. 14, 1 January 1985 (1985-01-01), pages 1-40, XP009086953, ISSN: 0065-2490
- KUSHNER DJ ET AL: "Pharmacological uses and perspectives of heavy water and deuterated compounds", CANADIAN JOURNAL OF PHYSIOLOGY AND PHARMACOLOGY, OTTAWA, ONT, CA, vol. 77, no. 2, 1 February 1999 (1999-02-01), pages 79-88, XP009086918,

## Description

### FIELD OF THE INVENTION

The invention relates to new deuterated pyrrolopyrimidine compounds and pharmaceutical compositions thereof, specifically deuterated pyrrolopyrimidine compounds and pharmaceutical compositions thereof which are inhibitors of CDK4/6. The invention is also directed to the use of these compounds and compositions in the treatment of hyperproliferative disorders, such as cancer.

### BACKGROUND OF THE INVENTION

Mammalian cell cycle progression is a tightly controlled process in which transitions through different phases are conducted in a highly ordered manner and guarded by multiple checkpoints. The retinoblastoma protein (pRb) is the checkpoint protein for the G1 to S phase transition. pRb associates with a family of E2F transcription factors to prevent their activity in the absence of appropriate growth stimuli *(See* Ortega et al. Biochimica et Biophysica Acta-Reviews on Cancer 2002; 1602 (1):73-87*;* Shapiro Journal of Clinical Oncology 2006; 24 (11):1770-1783*).* Upon mitogen stimulation, quiescent cells begin their entry into S phase by newly synthesizing D-cyclins, which are the activators of cyclin-dependent kinases 4 and 6 (CDK4/6). Once bound by the cyclins, CDK4/6 deactivate the pRb protein via phosphorylation. The phosphorylation of pRb releases E2F to direct the transcription of genes required for S phase. A full deactivation of pRb requires phosphorylations by both cyclin D-CDK4/6 and cyclin E-CDK2. Phosphorylations by CDK4/6 at specific sites of pRb (Ser780, Ser795) have been shown to be a prerequisite for cyclin E-CDK2 phosphorylation. *(See* Lundberg et al. Molecular and Cellular Biology 1998;18 (2):753-761) In addition to D-cyclins, the activity of CDK4/6 is regulated by p16, encoded by INK4a gene, which inhibits the kinase activity. *(See* Kamb et al. Science 1994; 264 (5157). 436-440*)* The CIP/KIP proteins, which are the inhibitors of cyclin E-CDK2, also bind to cyclin D-CDK4/6 complex, and this results in further activation of CDK2 by sequestering the CIP/KIP proteins away from their target. *(See* Sherr et al. Genes & Development 1999, 13 (12):1501-1512*)* Therefore, the cyclin D-CDK4/6 is a key enzyme complex that regulates the G1 to S phase.

The D-cyclin-CDK4/6-INK4a-pRb pathway is universally disrupted to favor cell proliferation in cancer. In a majority of cases (∼80%), cancers maintain a functional pRb and utilize different mechanisms to increase the activity of CDK4/6 kinase. *(See* Ortega et al. Biochimica et Biophysica Acta-Reviews on Cancer 2002; 1602 (1):73-87*;* Shapiro Joumal of Clinical Oncology 2006; 24 (11):1770-1783*))* In Mantle cell lymphoma (MCL), cyclin D1 is translocated to IgH promoter (t11:14) which results in constitutive expression of the protein, leading to activation of CDK4/6 *(See* Amin, et al. Archives of Pathology & Laboratory Medicine 2003; 127 (4):424-431; Oudat, et al. Modem Pathology 2001; 14 (1):175A) This translocation is observed in >90% of the MCL cases and considered pathognomic for the disease. The D-cyclin is also translocated in 20% of multiple myelomas. (See Bergsagel et al. Immunological Reviews 2003; 194 (1):96-104*)*

In addition to translocation, D-cyclin abundance can also be increased by amplification or overexpression, and the examples of these can be found in squamous cell esophageal cancer, where a significant portion exhibits cyclin D1 amplification (See Jiang, et al. Cancer Research 1992; 52 (10):2980-2983*)* and in breast cancer, where the overexpression of cyclin D1 is frequent (See Amold et al. Journal of Clinical Oncology 2005). The CDK4/6 kinase activity can also be increased by amplification of the CDK4 gene itself and the co-amplifications of CDK4 and MDM2 genes are observed in almost all cases of dedifferentiated liposarcomas. (See Sirvent, et al American Journal of Surgical Pathology 2007; 31 (10):1476-1489*)* The genetic inhibitor of CDK4/6 is also frequently inactivated in cancer to achieve CDK4/6 activation and the examples of this include non small cell lung cancer, melanoma and pancreatic cancer (Brambilla, et al Journal of Pathology 1999; 188 (4):351-360; Cowgill et al. American Journal of Surgery 2003; 186 (3):279-286*;* Gazzeri, et al Oncogene 1998; 16 (4):497-504*;* Kamb et al. Science 1994; 264 (5157):436-440; Ortega et al. Biochimica et Biophysica Acta-Reviews on Cancer 2002; 1602 (1):73-87*).*

In addition to these genetic defects directly related to the D-cyclin-CDK4/6-INK4a-pRb pathway, the activity of the CDK4/6 kinases can also be enhanced by oncogenic aberrations of the mitogen pathways that increase D-cyclin expression. The examples here include EGFR amplifications in non small cell lung cancer (NSCLC), activating K-Ras mutations in pancreatic cancer, V600E B-Raf mutation in melanoma and PTEN inactivation in colon cancer (See Dailey, et al Cytokine & Growth Factor Reviews 2005; 16 (2):233-247*;* Engelman Nature Reviews Cancer 2009; 9 (8):550-562; Garcia-Echeverria Purinergic Signalling 2009; 5 (1):117-125*,* Gray-Schopfer et al. Cancer and Metastasis Reviews 2005; 24 (1):165-183*,* John, et al. Oncogene 2009; 28:S14-S23*,* Sharma, et al Nature Reviews Cancer 2007; 7 (3):169-181*).*

Taken together, a large number of human neoplasms achieve enhanced cell proliferation by increasing CDK4/6 activity and a small molecule inhibitor of these kinases might provide an effective means to treat these diseases.

Inhibitors of CDKs are known and patent applications have been filed on such inhibitors. (See, for example, WO2007/14022 and WO2010/020675)

US 2009/203688, 13 August 2009, describes 7-phenyl-7H-pyrrolo[2,3d]pyrimidin-2yl-amino derivatives as inhibitors of the tyrosine kinase activity of Janus kinases.

MORIARTY K J Et. Al. "The Synthesis and SAR of 2-amino-pyrrolo[2,3-d] pyrimidines: A new class of Aurora-A kinase inhibitors", Bioorg. Med. Chem. Lett., 16, 2006, pages 5778-5783, describes certain pyrrolopyrimidine compounds as Aurora-A inhibitors.

NUTLEY BERNARD P Et. Al. "Metabolism and pharmacokinetics of the cyclin-dependent kinase inhibitor R-roscovitine in the mouse", Mol. Cancer. Ther., 4, 2005, pages 125-139, describes 2,6,9-trisubstituted purines as CDK inhibitors and metabolism studies thereof.

Thus attempts have been made to prepare compounds that inhibit CDK4/6 activity and a number of such compounds have been disclosed in the art. However, in view of the number of pathological responses that are mediated by CDK4/6, there remains a continuing need for inhibitors of CDK4/6 which can be used in the treatment of a variety of conditions, including cancer.

### SUMMARY OF THE INVENTION

The invention is directed to novel deuterated pyrrolopyrimidine compounds of formula (I) 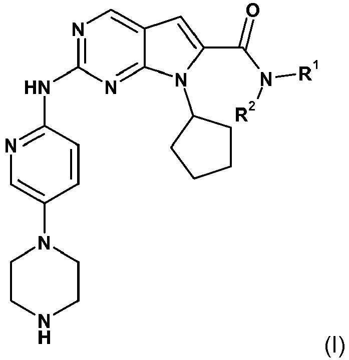 wherein R¹ and R² are defined below and to salts, including pharmaceutically acceptable salts thereof.

The compounds of the present invention are CDK4/6 inhibitors and could be useful in the treatment of diseases and disorders mediated by CDK4/6, such as cancer, including mantle cell lymphoma, liposarcoma, non small cell lung cancer, melanoma, squamous cell esophageal cancer and breast cancer. The invention is further directed to pharmaceutical compositions comprising a compound of the invention. The invention is still further directed to methods of inhibiting CDK4/6 activity and to the treatment of disorders associated therewith using a compound of the invention or a pharmaceutical composition comprising a compound of the invention.

### DETAILED DESCRIPTION OF THE INVENTION

A compound according to formula (I) 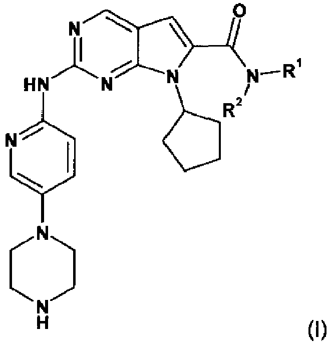 wherein:
R¹ is hydrogen, methyl, CH₂D, CHD₂, or CD₃; and
R² is CH₂D, CHD₂, or CD₃.

In one embodiment of the present invention R¹ is hydrogen, methyl, or CD₃; and R² is CD₃.

Specific compounds of formula (I) include:
7-Cyclopentyl-2-(5-piperzin-1-yl-pyridin-2-ylamino)-7H-pyrrolo[2,3-d]pyrimidine-6-carboxylic acid dimethylamide-d₆;
7-Cyclopentyl-2-(5-piperzin-1-yl-pyridin-2-ylamino)-7H-pyrrolo[2,3-d]pyrimidine-6-carboxylic acid methylamide-d₃; and
7-Cyclopentyl-2-(5-piperzin-1-yl-pyridin-2-ylamino)-7H-pyrrolo[2,3-d]pyrimidine-6-carboxylic acid methyl(methyl-d₃)amide.

### Terms and Definitions

"Deuterium", or "D", or "d" refers to an isotope of hydrogen whose nucleus contains one proton and one neutron. When a particular position is designated as having deuterium, it is understood that the abundance of deuterium at that position is greater than the natural abundance of deuterium (typically 0.015%). Unless otherwise stated, when a position is designated specifically as "D" or "deuterium", the position is understood to have deuterium at an abundance that is greater than the natural abundance of deuterium.

The term "compounds of the present invention" (unless specifically identified otherwise) refer to compounds of Formula (I) and salts thereof, including preferably pharmaceutically acceptable salts of the compounds.

As used herein the symbols and conventions used in the processes, schemes, and examples are consistent with those used in the contemporary scientific literature, for example the *Journal of the American Chemical Society* or the *Journal of Biological Chemistry.* Unless otherwise noted, all starting materials were obtained from commercial suppliers and used without further purification. Specifically the following abbreviations may be used in the examples and throughout the specification.
- boc: *tert*-butoxycarbonyl
- C: Celsius
- DMF: *N,N*-dimethylformamide
- DCE: dichloroethane
- DCM: dichloromethane
- DIPEA: diisopropylethylamine
- DMSO: dimethylsulfoxide
- EtOAc: ethyl acetate
- EtOH: ethanol
- g: grams
- h: hours
- HCl: hydrochloric acid
- LC: liquid chromatography
- LC/MS: liquid chromatography mass spectrum
- M: molar
- MeCN: acetonitrile
- MeOH: methanol
- MHz: megahertz
- Min.: minutes
- m/z: mass to charge
- N: normal
- NMR: nuclear magnetic resonance
- PEG(750): O-(2-aminoethyl)-O'-methyl polyethylene glycol 750; NH₂(CH₂CH₂O)ₙCH₃; CAS# [80506-64-5]; Fluka 07964; AVERAGE MW = 750
- MS: mass spectrum
- NMR: nuclear magnetic resonance

The skilled artisan will appreciate that salts, including pharmaceutically acceptable salts of the compounds of formula (I) may be prepared. As used herein, the terms "salt" or "salts" refers to an acid addition or base addition salt of a compound of the invention. The term "pharmaceutically acceptable salts" refers to salts that retain the biological effectiveness and properties of the compounds of this invention and, which typically are not biologically or otherwise undesirable. Accordingly the invention is further directed to salts, preferably pharmaceutically acceptable salts, of the compounds of formula (I).

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfornate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like. Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

The pharmaceutically acceptable salts of the present invention can be synthesized from a parent compound, a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, e.g., in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

Substitution with heavier isotopes, particularly deuterium (i.e., ²H or D) may afford certain therapeutic advantages resulting from greater metabolic stability, for example increased in vivo half-life or reduced dosage requirements or an improvement in therapeutic index. It is understood that deuterium in this context is regarded as a substituent of the compounds of the present invention. The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this invention is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

Furthermore, the compounds of the present invention, including their salts, can also be obtained in the form of their hydrates, or include other solvents used for their crystallization. The compounds of the present invention may inherently or by design form solvates with pharmaceutically acceptable solvents (including water); therefore, it is intended that the invention embrace both solvated and unsolvated forms. The term "solvate" refers to a molecular complex of a compound of the present invention (including pharmaceutically acceptable salts thereof) with one or more solvent molecules. Such solvent molecules are those commonly used in the pharmaceutical art, which are known to be innocuous to the recipient, e.g., water, ethanol, and the like. The term "hydrate" refers to the complex where the solvent molecule is water. Moreover, pharmaceutically acceptable solvates in accordance with the invention include those wherein the solvent of crystallization may be isotopically substituted, *e.g*. D₂O, d₆-acetone, d₆-DMSO.

Pro-drugs of the compounds of the present invention can convert *in vivo* to the compounds of the present invention. A pro-drug is an active or inactive compound that is modified chemically through *in vivo* physiological action, such as hydrolysis, metabolism and the like, into a compound of this invention following administration of the prodrug to a subject. The suitability and techniques involved in making and using pro-drugs are well known by those skilled in the art. Prodrugs can be conceptually divided into two non-exclusive categories, bioprecursor prodrugs and carrier prodrugs. See The Practice of Medicinal Chemistry, Ch. 31-32 (Ed. Wermuth, Academic Press, San Diego, Calif., 2001). Generally, bioprecursor prodrugs are compounds, which are inactive or have low activity compared to the corresponding active drug compound, that contain one or more protective groups and are converted to an active form by metabolism or solvolysis. Both the active drug form and any released metabolic products should have acceptably low toxicity.

Carrier prodrugs are drug compounds that contain a transport moiety, e.g., that improve uptake and/or localized delivery to a site(s) of action. Desirably for such a carrier prodrug, the linkage between the drug moiety and the transport moiety is a covalent bond, the prodrug is inactive or less active than the drug compound, and any released transport moiety is acceptably non-toxic. For prodrugs where the transport moiety is intended to enhance uptake, typically the release of the transport moiety should be rapid. In other cases, it is desirable to utilize a moiety that provides slow release, e.g., certain polymers or other moieties, such as cyclodextrins. Carrier prodrugs can, for example, be used to improve one or more of the following properties: increased lipophilicity, increased duration of pharmacological effects, increased site-specificity, decreased toxicity and adverse reactions, and/or improvement in drug formulation *(e.g.,* stability, water solubility, suppression of an undesirable organoleptic or physiochemical property). For example, lipophilicity can be increased by esterification of (a) hydroxyl groups with lipophilic carboxylic acids (e.g., a carboxylic acid having at least one lipophilic moiety), or (b) carboxylic acid groups with lipophilic alcohols (e.g., an alcohol having at least one lipophilic moiety, for example aliphatic alcohols).

Exemplary prodrugs are, *e.g*., esters of free carboxylic acids and S-acyl derivatives of thiols and *O*-acyl derivatives of alcohols or phenols, wherein acyl has a meaning as defined herein. Suitable prodrugs are often pharmaceutically acceptable ester derivatives convertible by solvolysis under physiological conditions to the parent carboxylic acid, *e.g.,* lower alkyl esters, cycloalkyl esters, lower alkenyl esters, benzyl esters, mono- or di-substituted lower alkyl esters, such as the ω-(amino, mono- or di-lower alkylamino, carboxy, lower alkoxycarbonyl)-lower alkyl esters, the α-(lower alkanoyloxy, lower alkoxycarbonyl or di-lower alkylaminocarbonyl)-lower alkyl esters, such as the pivaloyloxymethyl ester and the like conventionally used in the art. In addition, amines have been masked as arylcarbonyloxymethyl substituted derivatives which are cleaved by esterases *in vivo* releasing the free drug and formaldehyde (Bundgaard, J. Med. Chem. 2503 (1989)). Moreover, drugs containing an acidic NH group, such as imidazole, imide, indole and the like, have been masked with N-acyloxymethyl groups (Bundgaard, Design of Prodrugs, Elsevier (1985)). Hydroxy groups have been masked as esters and ethers. EP 039,051 (Sloan and Little) discloses Mannich-base hydroxamic acid prodrugs, their preparation and use.

### General Schemes

Scheme 1 illustrates the preparation of intermediate II which can be used in the preparation of compounds of the present invention.

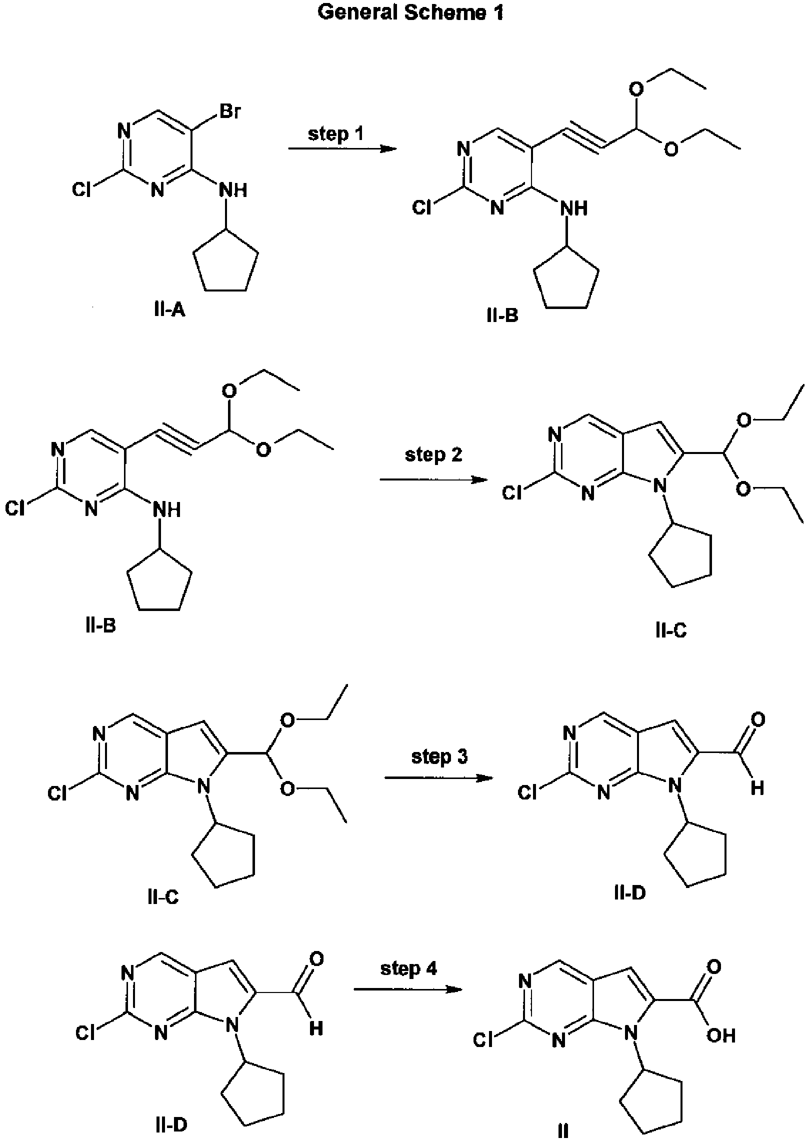

To form a compound of formula II-A, 5-bromo-2,4-dichloropyrimidine is added to a solvent, such as EtOH in a large sealed tube. An appropriate cycloalkyl-amine, such as cyclopentyl-amine and a base, such as DIPEA are added to the solution at room temperature. The solution is then stirred at room temperature overnight. The product may be purified by known methods to give a compound of formula II-A as shown above.

In step 1 of Scheme 1, a compound of formula II-A and propiolaldehydediethylacetel are combined by methods known in the art, e.g., a Sonogashira cross-coupling. See, e.g., Sonogashira, K., et al., Tetra Let, 16(50), 4467-4470 (1975). The compound of formula II-A and propiolaldehydediethylacetel are combined in basic conditions (e.g. with a commonly used base such as triethyl amine) with a palladium catalyst, such as PdCl₂(PPh₃)₂, and a copper catalyast, such as Cul, in a solvent, such as dimethylformamide (DMF), degassed and heated, e.g., to a temperature such as 100°C. After a period of time, such as 13 hours, the reaction product is purified by known methods to yield a compound of formula II-B.

In step 2 of Scheme 1, a mixture of a compound of formula II-B in a solvent, such as THF, is added 1 M tetra-n-butylammonium fluoride (TBAF) in THF and heated, to a temperature such as 65°C for a time, such as 2 hours. The reaction product is then purified to yield a compound of formula II-C.

In step 3 of Scheme 1, to a mixture of a compound of formula II-C in dioxane is added an acid, such as concentrated HCl. After reaction is completed within 10 min, water is added and then extracted with ethyl acetate. The reaction product is then purified to give an aldehyde of formula II-D.

In step 4 of Scheme 1, to a mixture of a compound of formula II-D in DMF is added oxone and stirred for 6 hours. After the reaction is complete, water is added and a yellow solid is precipitated and is collected by filtration to give a carboxylic acid of formula II.

### Compositions

In another aspect, the present invention provides a pharmaceutical composition comprising a compound of formula (I) or a pharmaceutically acceptable salt thereof and a pharmaceutically acceptable carrier or excipient. The pharmaceutical composition can be formulated for particular routes of administration such as oral administration, parenteral administration, and rectal administration, etc. In addition, the pharmaceutical compositions of the present invention can be made up in a solid form (including, without limitation, capsules, tablets, pills, granules, powders or suppositories), or in a liquid form (including, without limitation, solutions, suspensions or emulsions). The pharmaceutical compositions can be subjected to conventional pharmaceutical operations such as sterilization and/or can contain conventional inert diluents, lubricating agents, or buffering agents, as well as adjuvants, such as preservatives, stabilizers, wetting agents, emulsifers and buffers, etc.

Typically, the pharmaceutical compositions are tablets or gelatin capsules comprising the active ingredient together with a) diluents, *e.g*., lactose, dextrose, sucrose, mannitol, sorbitol, cellulose and/or glycine; b) lubricants, *e.g*., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and/or polyvinylpyrrolidone; if desired; d) disintegrants, *e.g*., starches, agar, alginic acid or its sodium salt, or effervescent mixtures; and/or e) absorbents, colorants, flavors and sweeteners.

Tablets may be either film coated or enteric coated according to methods known in the art. Suitable compositions for oral administration include an effective amount of a compound of the invention in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets may contain the active ingredient in admixture with nontoxic pharmaceutically acceptable excipients which are suitable for the manufacture of tablets. These excipients are, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffn or olive oil.

Certain injectable compositions are aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, or contain about 1-50%, of the active ingredient.

Suitable compositions for transdermal application include an effective amount of a compound of the invention with a suitable carrier. Carriers suitable for transdermal delivery include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

Suitable compositions for topical application, *e.g.*, to the skin and eyes, include aqueous solutions, suspensions, ointments, creams, gels or sprayable formulations, *e.g*., for delivery by aerosol or the like. Such topical delivery systems will in particular be appropriate for dermal application, *e.g*., for the treatment of skin cancer, *e.g*., for prophylactic use in sun creams, lotions, sprays and the like. They are thus particularly suited for use in topical, including cosmetic, formulations well-known in the art. Such may contain solubilizers, stabilizers, tonicity enhancing agents, buffers and preservatives.

As used herein a topical application may also pertain to an inhalation or to an intranasal application. They may be conveniently delivered in the form of a dry powder (either alone, as a mixture, for example a dry blend with lactose, or a mixed component particle, for example with phospholipids) from a dry powder inhaler or an aerosol spray presentation from a pressurised container, pump, spray, atomizer or nebuliser, with or without the use of a suitable propellant.

The present invention further provides anhydrous pharmaceutical compositions and dosage forms comprising the compounds of the present invention as active ingredients, since water may facilitate the degradation of certain compounds. Anhydrous pharmaceutical compositions and dosage forms of the invention can be prepared using anhydrous or low moisture containing ingredients and low moisture or low humidity conditions. An anhydrous pharmaceutical composition may be prepared and stored such that its anhydrous nature is maintained. Accordingly, anhydrous compositions are packaged using materials known to prevent exposure to water such that they can be included in suitable formulary kits. Examples of suitable packaging include, but are not limited to, hermetically sealed foils, plastics, unit dose containers (*e. g*., vials), blister packs, and strip packs.

The invention further provides pharmaceutical compositions and dosage forms that comprise one or more agents that reduce the rate by which the compound of the present invention as an active ingredient will decompose. Such agents, which are referred to herein as "stabilizers," include, but are not limited to, antioxidants such as ascorbic acid, pH buffers, or salt buffers, etc.

### Methods of Use

The compounds of the present invention are inhibitors of CDK4/6 and therefore may be capable of treating diseases wherein the underlying pathology is (at least in part) mediated by CDK4/6. Such diseases include cancer and other diseases in which there is a disorder of cell proliferation, apoptosis, or differentiation.

Thus, the compounds of the present invention may be useful in the treatment of RB+ve (retinoblastoma protein positive) tumours, including tumours harbouring mutations in Ras, Raf, Growth Factor Receptors or over-expression of Growth Factor Receptors. The compounds of the present invention may also be useful in the treatment of tumours with amplifications of CDK4 and CDK6 genes as well as, tumours over-expressing cyclin partners of the cyclin dependent kinases. The compounds of the present invention may also be useful in the treatment of RB-ve tumours.

The compounds of the present invention may also be useful in the treatment tumours with genetic aberrations that activate the CDK4/6 kinase activity. These include, but are not limited to, cancers with D-cyclin translocations such as mantle cell lymphoma and multiple myeloma, D-cyclin amplifications such as breast cancer and squamous cell esophageal cancer, CDK4 amplifications such as liposarcoma, CDK6 amplifications or overexpressions such as T-cell lymphoma and p16 inactivation such as melanoma, non-small cell lung cancer and pancreatic cancer.

The compounds of the present invention may be useful in the treatment of cancers that have genetic aberrations in the upstream regulators of D-cyclins, where the defect results in an increase of D-cyclins abundance, can also be considered for treatment. These include, but are not limited to, acute myeloid leukemia with FLT3 activation, breast cancers with Her2/neu overexpression, ER dependency or triple negative phenotype, colon cancers with activating mutations of the MAPK, PI3K or WNT pathway, melanomas with activating mutations of MAPK pathway, non small cell lung cancers with activating aberrations of EGFR pathway and pancreatic cancers with activating aberrations of MAPK pathway including K-ras mutations.

Examples of cancers which may be treated with a compound of the present invention include but are not limited to, carcinoma, for example a carcinoma of the bladder, breast, colon (e.g. colorectal carcinomas such as colon adenocarcinoma and colon adenoma), kidney, epidermis, liver, lung(e.g. adenocarcinoma, small cell lung cancer and non-small cell lung carcinomas), oesophagus, gall bladder, ovary, pancreas (e.g. exocrine pancreatic carcinoma), stomach, cervix, thyroid, nose, head and neck, prostate, and skin (e.g. squamous cell carcinoma). Other examples of cancers that may be treated with a compound of the present invention include hematopoietic tumours of lymphoid lineage (e.g. leukemia, acute lymphocytic leukemia, mantle cell lymphoma, chronic lymphocytic leukaemia, B-cell lymphoma,(such as diffuse large B cell lymphoma), T-cell lymphoma, multiple myeloma, Hodgkin's lymphoma, non-Hodgkin's lymphoma, hairy cell lymphoma, and Burkett's lymphoma; hematopoietic tumours of myeloid lineage, for example acute and chronic myelogenous leukemias, myelodysplastic syndrome, and promyelocytic leukemia. Other cancers include thyroid follicular cancer; a tumour of mesenchymal origin, for example fibrosarcoma or habdomyosarcoma; a tumour of the central or peripheral nervous system, for example astrocytoma, neuroblastoma, glioma or schwannoma; melanoma; seminoma; teratocarcinoma; osteosarcoma; xeroderma pigmentosum; retinoblastoma; keratoctanthoma; thyroid follicular cancer; and Kaposi's sarcoma.

One group of cancers includes human breast cancers (e.g. primary breast tumours, node-negative breast cancer, invasive duct adenocarcinomas of the breast, non-endometrioid breast cancers); and endometrial cancers. Another sub-set of cancers wherein compounds having CDK4/6 inhibitory activity may be of particular therapeutic benefit comprises glioblastoma multiforme, T cell ALL, sarcomas, familial melanoma and melanoma.

CDK4/6 inhibitors could also be useful in the treatment of viral infections, for example herpes virus, pox virus, Epstein-Barr virus, Sindbis virus, adenovirus, HIV, HPV, HCV and HCMV; prevention of AIDS development in HIV-infected individuals; chronic inflammatory diseases, for example systemic lupus erythematosus, autoimmune mediated glomerulonephritis, rheumatoid arthritis, psoriasis, inflammatory bowel disease, and autoimmune diabetes mellitus; cardiovascular diseases for example cardiac hypertrophy, restenosis, atherosclerosis; neurodegenerative disorders, for example Alzheimer's disease, AIDS-related dementia, Parkinson's disease, amyotropic lateral sclerosis, retinitis pigmentosa, spinal muscular atropy and cerebellar degeneration; glomerulonephritis; myelodysplastic syndromes, ischemic injury associated myocardial infarctions, stroke and reperfusion injury, arrhythmia, atherosclerosis, toxin-induced or alcohol related liver diseases, haematological diseases, for example, chronic anemia and aplastic anemia; degenerative diseases of the musculoskeletal system, for example, osteoporosis and arthritis, aspirin-senstive rhinosinusitis, cystic fibrosis, multiple sclerosis, kidney diseases, ophthalmic diseases including age related macular degeneration, uveitis, and cancer pain.

Methods of treatment comprise administering an effective amount of a compound according to formula (I), or a pharmaceutically acceptable salt thereof, to a subject in need thereof. Compounds of the invention can be used in methods of treating any one of the above mentioned disorders or diseases by administering an effective amount of a compound according to formula (I), or a pharmaceutically acceptable salt thereof, to a subject in need thereof.

The term "a therapeutically effective amount" of a compound of the present invention refers to an amount of the compound of the present invention that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the compound of the present invention that, when administered to a subject, is effective to (1) at least partially alleviating, inhibiting, preventing and/or ameliorating a condition, or a disorder or a disease (i) mediated by CDK4/6, or (ii) associated with CDK4/6 activity, or (iii) characterized by activity (normal or abnormal) of CDK4/6; or (2) reducing or inhibiting the activity of CDK4/6; or (3) reducing or inhibiting the expression of CDK4/6. In another non-limiting embodiment, the term "a therapeutically effective amount" refers to the amount of the compound of the present invention that, when administered to a cell, or a tissue, or a non-cellular biological material, or a medium, is effective to at least partially reducing or inhibiting the activity of CDK4/6; or at least partially reducing or inhibiting the expression of CDK4/6. The meaning of the term "a therapeutically effective amount" as illustrated in the above embodiment for CDK4/6 also applies by the same means to any other relevant proteins/peptides/enzymes.

As used herein, the term "subject" refers to an animal. Typically the animal is a mammal. A subject also refers to for example, primates (e.g., humans, male or female), cows, sheep, goats, horses, dogs, cats, rabbits, rats, mice, fish, birds and the like. In certain embodiments, the subject is a primate. In yet other embodiments, the subject is a human.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both. In yet another embodiment, "treat", "treating" or "treatment" refers to preventing or delaying the onset or development or progression of the disease or disorder.

As used herein, a subject is "in need of" a treatment if such subject would benefit biologically, medically or in quality of life from such treatment.

The pharmaceutical composition or combination of the present invention can be in unit dosage of about 1-1000 mg of active ingredient(s) for a subject of about 50-70 kg, or about 1-500 mg or about 1-250 mg or about 1-150 mg or about 0.5-100 mg, or about 1-50 mg of active ingredients. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The above-cited dosage properties are demonstrable *in vitro* and *in vivo* tests using advantageously mammals, e.g., mice, rats, dogs, monkeys or isolated organs, tissues and preparations thereof. The compounds of the present invention can be applied in *vitro* in the form of solutions, e.g., aqueous solutions, and *in vivo* either enterally, parenterally, advantageously intravenously, e.g., as a suspension or in aqueous solution. The dosage *in vitro* may range between about 10⁻³ molar and 10⁻⁹ molar concentrations. A therapeutically effective amount *in vivo* may range depending on the route of administration, between about 0.1-500 mg/kg, or between about 1-100 mg/kg.

Another embodiment of the present invention is a compound of formula (I), or a pharmaceutically acceptable salt thereof, for use in the treatment of a disorder or disease mediated by CDK4/6.

Another embodiment of the present invention is a compound of formula (I), or a pharmaceutically acceptable salt thereof, for use in the treatment of a disorder or a disease mediated by CDK4/6, in a subject wherein the disorder or the disease is selected from the group consisting of mantle cell lymphoma, multiple myeloma, breast cancer, squamous cell esophageal cancer, liposarcoma, T-cell lymphoma melanoma, non small cell lung cancer, and pancreatic cancer.

Another embodiment of the present invention is a compound according to formula (I), or a pharmaceutically acceptable salt thereof, for use as a medicament.

Another embodiment of the present invention is the use of a compound of formula (I), or a pharmaceutically acceptable salt thereof, in the manufacture of a medicament for the treatment of a disorder or disease mediated by CDK4/6 wherein the disorder or the disease is selected from the group consisting of mantle cell lymphoma, multiple myeloma, breast cancer, squamous cell esophageal cancer, liposarcoma, T-cell lymphoma melanoma, non small cell lung cancer, and pancreatic cancer.

### Combinations

The compounds of the present invention may be administered either simultaneously with, or before or after, one or more other therapeutic agents. The compounds of the present invention may be administered separately, by the same or different route of administration, or together in the same pharmaceutical composition as the other agents.

In one embodiment, the invention provides a product comprising a compound of formula (I), or a pharmaceutically acceptable salt thereof, and at least one other therapeutic agent as a combined preparation for simultaneous, separate or sequential use in therapy. In one embodiment, the therapy is the treatment of a disease or condition mediated by CDK4/6 inhibition. Products provided as a combined preparation include a composition comprising the compound of the present invention and the other therapeutic agent(s) together in the same pharmaceutical composition, or the compound of the present invention and the other therapeutic agent(s) in separate form, e.g. in the form of a kit.

In one embodiment, the invention provides a pharmaceutical composition comprising a compound of formula (I) or a pharmaceutically acceptable salt thereof, and another therapeutic agent(s). Optionally, the pharmaceutical composition may comprise a pharmaceutically acceptable excipient, as described above.

In one embodiment, the invention provides a kit comprising two or more separate pharmaceutical compositions, at least one of which contains a compound of formula (I) or a pharmaceutically acceptable salt thereof. In one embodiment, the kit comprises means for separately retaining said compositions, such as a container, divided bottle, or divided foil packet. An example of such a kit is a blister pack, as typically used for the packaging of tablets, capsules and the like.

The kit of the invention may be used for administering different dosage forms, for example, oral and parenteral, for administering the separate compositions at different dosage intervals, or for titrating the separate compositions against one another. To assist compliance, the kit of the invention typically comprises directions for administration.

In the combination therapies of the invention, the compound of the invention and the other therapeutic agent may be manufactured and/or formulated by the same or different manufacturers. Moreover, the compound of the invention and the other therapeutic may be brought together into a combination therapy: (i) prior to release of the combination product to physicians (e.g. in the case of a kit comprising the compound of the invention and the other therapeutic agent); (ii) by the physician themselves (or under the guidance of the physician) shortly before administration; (iii) in the patient themselves, e.g. during sequential administration of the compound of the invention and the other therapeutic agent.

Accordingly, the invention provides a compound of formula (I), or a pharmaceutically acceptable salt thereof, for use in treating a disease or condition mediated by inhibition of CDK4/6, wherein the medicament is prepared for administration with another therapeutic agent. The invention also provides another therapeutic agent for use in treating a disease or condition mediated by inhibition of CDK4/6, wherein the medicament is administered with a compound of the present invention.

The invention also provides a compound of formula (I), or a pharmaceutically acceptable salt thereof, for use in a method of treating a disease or condition mediated by CDK4/6 inhibition, wherein the compound of formula (I) is prepared for administration with another therapeutic agent. The invention also provides another therapeutic agent for use in a method of treating a disease or condition mediated by CDK4/6 inhibition, wherein the other therapeutic agent is prepared for administration with a compound of formula (I) or a pharmaceutically acceptable salt thereof. The invention also provides a compound of formula (I), or a pharmaceutically acceptable salt thereof, for use in a method of treating a disease or condition mediated by CDK4/6 inhibition, wherein the compound of formula (I), or a pharmaceutically acceptable salt thereof, is administered with another therapeutic agent. The invention also provides another therapeutic agent for use in a method of treating a disease or condition mediated by CDK4/6 inhibition, wherein the other therapeutic agent is administered with a compound of formula (I).

The invention also provides a compound of formula (I), or a pharmaceutically acceptable salt thereof, for use in treating a disease or condition mediated by CDK4/6, wherein the patient has previously (e.g. within 24 hours) been treated with another therapeutic agent. The invention also provides another therapeutic agent for use in treating a disease or condition mediated by CDK4/6, wherein the patient has previously (e.g. within 24 hours) been treated with a compound of formula (I) or a pharmaceutically acceptable salt thereof.

In one embodiment, the other therapeutic agent is selected from an anti-inflammatory, antiproliferative, chemotherapeutic agent, immunosuppressant, anti-cancer, cytotoxic agent or kinase inhibitor other than a compound of the present invention, or salt thereof. Further examples of agents that may be administered in combination with the compounds of the invention include, but are not limited to, a PTK inhibitor, cyclosporin A, CTLA4-Ig, antibodies selected from anti-ICAM-3, anti-IL-2 receptor, anti-CD45RB, anti-CD2, anti-CD3, anti-CD4, anti-CD80, anti-CD86, and monoclonal antibody OKT3, agents blocking the interaction between CD40 and gp39, fusion proteins constructed from CD40 and gp39, inhibitors of NF-kappa B function, non-steroidal antiinflammatory drugs, steroids, gold compounds, antiproliferative agents, FK506, mycophenolate mofetil, cytotoxic drugs, TNF-α inhibitors, anti-TNF antibodies or soluble TNF receptor, rapamycin, mTor inhibitors, leflunimide, cyclooxygenase-2 inhibitors, paclitaxel, cisplatin, carboplatin, doxorubicin, carminomycin, daunorubicin, aminopterin, methotrexate, methopterin, mitomycin C, ecteinascidin 743, porfiromycin, 5-fluorouracil, 6-mercaptopurine, gemcitabine, cytosine arabinoside, podophyllotoxin, etoposide, etoposide phosphate, teniposide, melphalan, vinblastine, vincristine, leurosidine, epothilone, vindesine, leurosine, B-Raf inhibitor, MEK inhibitor, PI3K inhibitor, HSP90 inhibitor, CDK1 inhibitor, CDK2 inhibitor, CDK5 inhibitor, CDK7 inhibitor, CDK8 inhibitor, CDK9 inhibitor, EGFR inhibitor, FGFR inhibitor, PDGFR inhibitor, Her2/neu inhibitor, FLT3 inhibitor, Antagonists of androgen, glucocorticoid and prosterone receptors, SMO inhibitor, WNT inhibitor, Bcl inhibitor, IAP inhibitor, Mcl inhibitor, MDM2 inhibitor, p52 inhibitor, proteosome inhibitors (Velcade), or derivatives thereof.

Specific individual combinations which may provide particular treatment benefits include co-treatment of mantle cell lymphoma or pancreatic cancer patients with mTOR inhibitors, such as Everolimus.

A compound of the present invention may also be used in combination with other agents, e.g., an additional protein kinase inhibitor that is or is not a compound of the invention, for treatment of a protein kinase-associated disorder in a subject. By the term "combination" is meant either a fixed combination in one dosage unit form, or a kit of parts for the combined administration where a compound of the present invention and a combination partner may be administered independently at the same time or separately within time intervals that especially allow that the combination partners show a cooperative, *e.g*., synergistic, effect, or any combination thereof.

The compounds of the invention may be administered, simultaneously or sequentially, with an antiinflammatory, antiproliferative, chemotherapeutic agent, immunosuppressant, anti-cancer, cytotoxic agent or kinase inhibitor other than a compound of the Formula I or pharmaceutically acceptable salt thereof. Further examples of agents that may be administered in combination with the compounds of the invention include, but are not limited to, a PTK inhibitor, cyclosporin A, CTLA4-Ig, antibodies selected from anti-ICAM-3, anti-IL-2 receptor, anti-CD45RB, anti-CD2, anti-CD3, anti-CD4, anti-CD80, anti-CD86, and monoclonal antibody OKT3, agents blocking the interaction between CD40 and gp39, fusion proteins constructed from CD40 and gp39, inhibitors of NF-kappa B function, non-steroidal antiinflammatory drugs, steroids, gold compounds, antiproliferative agents, FK506, mycophenolate mofetil, cytotoxic drugs, TNF-α inhibitors, anti-TNF antibodies or soluble TNF receptor, rapamycin, leflunimide, cyclooxygenase-2 inhibitors, paclitaxel, cisplatin, carboplatin, doxorubicin, carminomycin, daunorubicin, aminopterin, methotrexate, methopterin, mitomycin C, ecteinascidin 743, porfiromycin, 5-fluorouracil, 6-mercaptopurine, gemcitabine, cytosine arabinoside, podophyllotoxin, etoposide, etoposide phosphate, teniposide, melphalan, vinblastine, vincristine, leurosidine, epothilone, vindesine, leurosine, or derivatives thereof.

A compound of the invention and any additional agent may be formulated in separate dosage forms. Alternatively, to decrease the number of dosage forms administered to a patient, the compound of the invention and any additional agent may be formulated together in any combination. For example, the compound of the invention inhibitor may be formulated in one dosage form and the additional agent may be formulated together in another dosage form. Any separate dosage forms may be administered at the same time or different times.

Alternatively, a composition of this invention comprises an additional agent as described herein. Each component may be present in individual compositions, combination compositions, or in a single composition.

### Examples

### Example 1: Synthesis of 7-cyclopentyl-2-(5-piperzin-1-yl-pyridin-2-ylamino)-7H-pyrrolo[2,3-d]pyrimidine-6-carboxylic acid dimethylamide-d₆.

### Step 1: Synthesis of 2-chloro-7-cyclopentyl-7H-pyrrolo[2,3-d]pyrimidine-6-carboxylic acid dimethylamide-d_{6.}

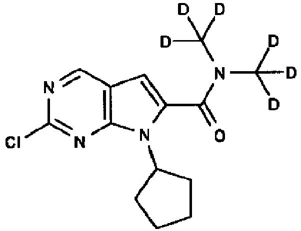

To a solution of 50 mmol (13.3 g) of 2-chloro-7-cyclopentyl-7H-pyrrolo[2,3-d]pyrimidine-6-carboxylic acid in 250 ml DMF was added 75 mmol (6.6 g) of dimethyl-d₆-amine hydrochloride, 55 mmol (20.9 g) of 2-(1H-7-azabenzotriazol-1-yl)-1,1,3,3-tetramethyluronium hexafluorophosphate, and 150 mmol (26.2 mL) of *N,N-*diisopropylethylamine. After stirring overnight at 25°C the reaction was diluted with EtOAc, washed with brine, dried over Na₂SO₄, filtered and concentrated. The residue was purified by silica gel chromatography using an EtOAc/heptane gradient to yield 30.6 mmol (9.7 g) of the title compound as a light pink solid. MS m/z 299.5 (M+H)⁺.

### Step 2: Synthesis of 4-[6-(7-cyclopentyl-6-dimethyl-d₆-carbamoyl-7H-pyrrolo[2,3-d]pyrimidin-2-ylamino)-pyridin-3-yl]-piperazine-1-carboxylic acid tert-butyl ester.

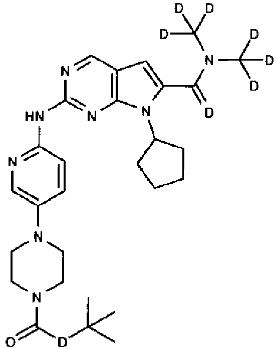

In a dry 250 ml flask was added 11.4 mmol (3.4 g) of 2-chloro-7-cyclopentyl-7H-pyrrolo[2,3-d]pyrimidine-6-carboxylic acid dimethylamide-d₆, 12.0 mmol (3.3 g) of 4-(6-amino-pyridin-3-yl)-piperazine-1-carboxylic acid tert-butyl ester, 17.1 mmol (5.6 g) of cesium carbonate, 0.57 mmol (0.35 g) of 2,2'-bis(diphenylphosphino)-1,1'-binaphthalene, and 0.57 mmol (0.13 g) of palladium acetate. The flask was sealed, evacuated and purged with dry nitrogen three times before adding 60 ml 1,4-dioxane. The reaction mixture was heated to 95°C for 4 hours in an oil bath. After cooling the reaction was diluted with 100 mL heptane, stirred for 30 min. and filtered. The recovered solid was suspended in water with vigorous stirring, sonicated for 5 min, and reisolated by filtration to give a brown solid after drying. The residue was purified by silica gel chromatography using a MeOH/EtOAc gradient to give a light brown solid. The solid was dissolved in 40 mL DCM and stirred for 16 hours with 1 g of Pd scavenger MP-TMT. After filtering and concentrating 9.5 mmol (5.1 g) of the title compound was recovered as a light brown solid. MS m/z 521.7 (M+H)⁺.

### Step 3: Synthesis of the title compound.

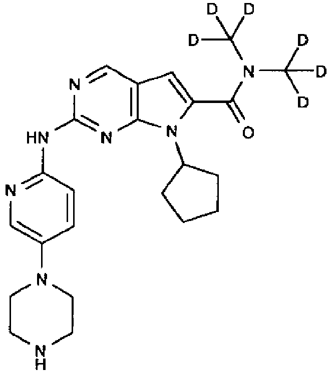

To a suspension of 9.5 mmol (5.1 g) of 4-[6-(7-cyclopentyl-6-dimethyl-d₆-carbamoyl-7H-pyrrolo[2,3-d]pyrimidin-2-ylamino)-pyridin-3-yl]-piperazine-1-carboxylic acid tert-butyl ester in 10 mL toluene was added slowly 1 mL of 6 N HCl. After stirring at 25°C for 1 hour, an additional 16 mL of 1 N HCl was added and stirred for 15 min. The reaction mixture was filtered through a pad of celite and the two phases were separated. The aqueous phase was slowly adjusted to pH 9 with 50% concentrated NaOH and resulting suspension was stirred at 25°C for 3 hour. The solid that was recovered by filtration was washed with water several times and triturated with 2-propanol. The result solid was dried under vacuo at 60°C for 16 hour to give 3.8 mmol (1.67 g) of the title compound as a tan-colored solid. ¹H NMR (400 MHz, DMSO): δ 9.55 (s, 1 H), 8.79 (s, 1 H), 8.20 (d, J = 8 Hz, 1 H), 8.06 (d, J = 4 Hz, 1 H), 7.48 (dd, J = 8 Hz, 4 Hz, 1 H), 6.61 (s, 1 H), 4.79-4.70 (m, 1 H), 3.25-3.23 (m, 4 H), 3.12-3.09 (m, 4 H), 2.47-2.42 (m, 2 H), 1.99 (br s, 4 H), 1.65 (br s, 2 H). ¹³C NMR (400 MHz, DMSO): δ 162.84, 154.67, 152.10, 151.11, 146.35, 142.19, 135.80, 131.80, 125.52, 112.52, 111.75, 100.60, 56.88, 48.31, 44.27, 29.66, 24.16; HRMS calcd for C23H24D6N80.H⁺ (M+H)⁺ 441.2997, found 441.3008.

### Example 2: Synthesis of 7-cyclopentyl-2-(5-piperzin-1-yl-pyridin-2-ylamino)-7H-pyrrolo[2,3-d]pyrimidine-6-carboxylic acid methylamide-d₃.

### Step 1: Synthesis of 2-chloro-7-cyclopentyl-7H-pyrrolo[2,3-d]pyrimidine-6-carboxylic acid methylamide-d₃.

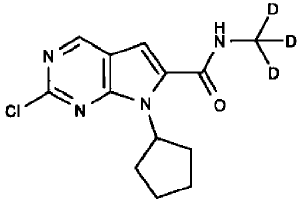

To a solution of 10 mmol (2.7 g) of 2-chloro-7-cyclopentyl-7H-pyrrolo[2,3-d]pyrimidine-6-carboxylic acid in 5 mL CH₂Cl₂ was added 15 mmol (1.3 mL) of oxalyl chloride followed by a few drops of DMF. The reaction was stirred overnight at room temperature and then concentrated under reduced pressure. The crude oil was redissolved in 100 mL CH₂Cl₂ in a 250 mL single neck flask. After the addition of 5 mmol diisopropylethylamine (0.87 mL), the flask was sealed with a rubber balloon. Methylamine-d₅ (50 mmol) as a gas was released into the sealed flask while being cooling to -40°C. After all the gas was introduced the reaction was warmed to room temperature. After stirring overnight at 25°C the reaction was diluted with CH₂Cl₂, washed with brine, dried over Na₂SO₄, filtered and concentrated. The residue was purified by silica gel chromatography using a EtOAc/heptane gradient from 15 to 100% to yield 5.4 mmol (1.5 g) of the title compound as an orange solid. MS m/z 282.4 (M+H)⁺.

### Step 2: Synthesis of 4-[6-(7-Cyclopentyl-6-methyl-d₃-carbamoyl-7H-pyrrolo[2,3-d]pyrimidin-2-ylamino)-pyridin-3-yl]-piperazine-1-carboxylic acid tert-butyl ester.

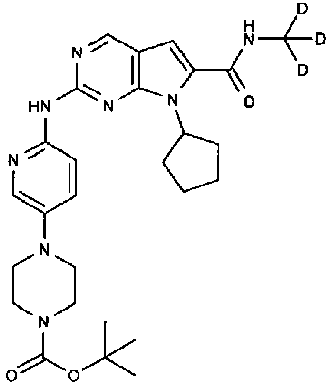

In a dry 20 ml microwave tube was added 5.4 mmol (1.5 g) of 2-chloro-7-cyclopentyl-7H-pyrrolo[2,3-d]pyrimidine-6-carboxylic acid methylamide-d₃, 5.4 mmol (1.5 g) of 4-(6-amino-pyridin-3-yl)-piperazine-1-carboxylic acid tert-butyl ester, 8.0 mmol (2.6 g) of cesium carbonate, 0.27 mmol (0.17 g) of 2,2'-bis(diphenylphosphino]-1,1'-binaphthalene and 1,4-dioxane (20 mL). The suspension was degassed by bubbling N₂ for 3 min before adding 0.57 mmol (0.06 g) of palladium acetate. The sealed tube was heated to 120°C for 20 min in a microwave reactor. After cooling the reaction was diluted with 100 mL heptane, stirred for 30 min. and filtered. The recovered solid was suspended in water with vigorous stirring, sonicated for 5 min, and re-isolated by filtration to give 3.1 mmol (1.6 g) of the title compound as a yellow solid. MS m/z 524.7 (M+H)⁺.

### Step 3: Synthesis of the title compound.

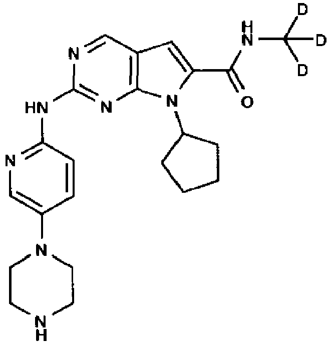

By using the general procedure described in Example 1, Step 3 using the appropriate starting materials, 1.94 mmol (0.82 g) of the title compound was isolated as a grey-colored solid. ¹H NMR (400 MHz, DMSO): δ 9.46 (s, 1 H), 8.83 (s, 1 H), 8.51 (s, 1 H), 8.13 (d, *J* = 8 Hz, 1 H), 8.00 (d, *J* = 4 Hz, 1 H), 7.41 (dd, *J =* 8 Hz, 4 Hz, 1 H), 6.89 (s, 1 H), 5.58-5.49 (m, 1 H), 3.04-3.01 (m, 4 H), 2.86-2.83 (m, 4 H), 2.51-2.44 (m, 2 H), 2.04-1.88 (m, 4 H), 1.68-1.63 (m, 2 H). ¹³C NMR (400 MHz, DMSO): δ 162.06, 154.98, 152.85, 151.88, 145.73, 143.02, 135.43, 131.75, 125.02, 112.75, 111.18, 102.79, 55.96, 49.87, 45.48, 29.42, 24.21; HRMS calcd for C22H25D3N8O.H⁺ (M+H)⁺ 424.2652, found 424.2650.

### Example 3: Synthesis of 7-cyclopentyl-2-(5-piperzin-1-yl-pyridin-2-ylamino)-7H-pyrrolo[2,3-d]pyrimidine-6-carboxylic acid methyl(methyl-d₃)amide.

### Step 1: Synthesis of 2-chloro-7-cyclopentyl-7H-pyrrolo[2,3-d]pyrimidine-6-carboxylic acid methyl(methyl-d₃)amide.

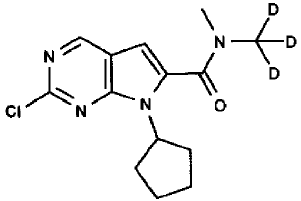

By using the general procedure described in Example 1, Step 1 using the appropriate starting materials, 1.25 mmol (0.39 g) of the title compound was isolated as a light pink solid. MS m/z 296.4 (M+H)⁺.

### Step 2: Synthesis of 4-[6-(7-cyclopentyl-6-methyl(methyl-d₃)-carbamoyl-7H-pyrrolo[2,3-d]pyrimidin-2-ylamino)-pyridin-3-yl]-piperazine-1-carboxylic acid tert-butyl ester.

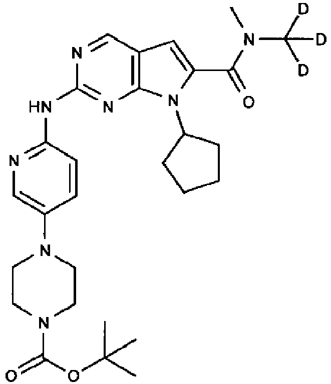

By using the general procedure described in Example 1, Step 2 using the appropriate starting materials, 0.30 mmol (0.16 g) of the title compound was isolated as a light yellow solid. MS m/z 538.7 (M+H)⁺.

### Step 3: Synthesis of the title compound: 7-cyclopentyl-2-(5-piperzin-1-yl-pyridin-2-ylamino)-7H-pyrrolo[2,3-d]pyrimidine-6-carboxylic acid methyl(methyl-d₃)amide.

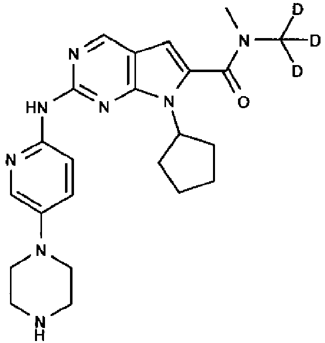

By using the general procedure described in Example 1, Step 3 using the appropriate starting materials, 0.17 mmol (0.08 g) of the title compound was isolated as a tan-colored solid. ¹H NMR (400 MHz, DMSO): δ 9.43 (s, 1 H), 8.77 (s, 1 H), 8.15 (d, J = 8 Hz, 1 H), 8.00 (s, 1 H), 7.41 (d, J = 8 Hz, 1 H), 6.60 (s, 1 H), 4.73 (br s, 1 H), 3.03 (br s, 7 H), 2.85 (br s, 4 H), 2.44 (br s, 2 H), 1.98 (br s, 4 H), 1.64 (br s, 2 H). ¹³C NMR (400 MHz, DMSO): δ 162.85, 154.73, 152.08, 151.18, 145.90, 142.93, 135.46, 131.72, 125.11, 112.58, 111.68, 100.59, 56.87, 49.89, 45.48, 29.66, 24.16; HRMS calcd for C23H27D3N8O.H⁺ (M+H)⁺ 438.2809, found 438.2813.

### Biological Data

### CDK4/cyclin D1 and CDK6/cyclin D3 Enzymatic Activity Assay

A 384-well microtiter Lance TR-FRET (time-resolved - fluorescence energy transfer) endpoint assay was used for CDK4/cyclin D1 and **CDK6/cyclin** D3 kinase activity measurements. The same assay was used for IC50 determination of small molecule inhibitors. In general, the kinase reactions were carried out in 30 µL volumes in the reaction solution containing the following: 2 uL compound (in 20% DMSO), 18 uL CDK4/cyclin D1 in Assay Buffer (50 mM HEPES, pH 7.5, 5 mM MgCl₂, 2 mM MnCl₂, 1 mM DTT, 0.05% BSA, 0.02% Tween-20), 10 uL of the mixture of pRb152 and ATP. The final reaction mixture contains compound (inhibitor) with the concentration varying from 0.005 - 10 µM, 2% DMSO, 0.3 nM CDK4/cyclin D1 or CDK6/cyclin D3, 175 nM pRb152, and 3 µM ATP (Amersham Pharmacia, Cat. No. 27-2056-01). All reactions were run at room temperature in 384-well white flat-bottom OptiPlates (Perkin Elmer, Cat. No. 6007290) for 60 min then were quenched by the addition of 10 µL of 120 mM EDTA. The signals were captured by the addition of 40 µL of the Detection Solution containing the following: Detection Buffer (50 mM HEPES, pH 7.5, 30 mM EDTA, 0.1% Triton x-100, 0.05% BSA), 70 ng/mL anti-phospho-pRb(S780) (Cell Signaling Technology, Cat. No. 9307S), 1 nM Lance Eu-W1024-Rabbit anti-IgG (Perkin Elmer, Cat. No. AD0082), and 20 nM SureLight™ Allophycocyanin-Streptavidin (Perkin Elmer, Cat. No. CR130-100). The resulted solutions were incubated at room temperature for 2 hours before read on the Evision Multilabel Reader (Perkin Elmer, Envision 2102-0010). Note: IC₅₀ < 0.005 nM or IC₅₀ > 10 µM indicates the true IC₅₀ is out of detection range.

CDK4/cyclin D1 recombinant protein used in the enzymatic activity assay was prepared by coexpressing pDEST10-CDK4 (N-terminal His₆) and pFastBacDual-GST-hCyclinD1 viruses in Sf21 cells. The overexpressed protein was purified by Ni-NTA affinity pull down to >80% pure by Sizing HPLC. CDK6/cyclin D3 enzyme complex was purchased from a commercial source (CarnaBiosciences, Cat. No. 04-107)

### CDK1/cyclin B Enzymatic Activity Assay

A 384-well microtiter IMAP-FP™ (Molecular Devices Trade Mark Technology) endpoint assay was used for CDK1/cyclin B kinase activity measurements. The same assay was used for IC₅₀ determination of small molecue inhibitors. In general, the kinase reactions were carried out in 20 µL volumes in the reaction solution, which is composed of 2 µL compound (in 20% DMSO), 8 µL CDK1/cyclin B in the 1x Reaction Buffer (Molecular Devices, Cat. No. R8139), 10 µL substrate mixture of Tamra Histone-H1 peptide (Molecular Devices, Cat. No. R7384) and ATP (Amersham Pharmacia, Cat. No. 27-2056-01) in the 1x Reaction Buffer with 1 mM DTT freshly added. The final reaction mixture contains compound (inhibitor) with the concentration varying from 0.005 - 10 µM, 2% DMSO, 0.25 nM CDK1/cyclin B, 100 nM Tamra Histone-H1 peptide, and 20 µM ATP.

All reactions were run at room temperature in black 384-well flat-bottom Costar plates (Corning, Cat. No. 3710) for 120 min then were quenched by the addition of 60 µL 400-fold diluted 1x Progressive Binding Buffer A (Molecular Devices, Cat. No. R8139). The fluorescent polarization signals were read on the Evision Multilabel Reader (Perkin Elmer, Envision 2102-0010) after 2-hour incubation at room temperature. Note: IC₅₀ < 0.005 nM or IC₅₀ > 10 µM indicates the true IC₅₀ is out of detection range.

**TABLE 1. BIOLOGICAL ACTIVITY**

| **Example #** | **CDK4 (µM)** | **CDK1 (µM)** |
|---|---|---|
| 1 | 0.025 | >15 |
| 2 | 0.132 | >15 |
| 3 | 0.015 | >15 |

### Comparators

The present invention includes deuterated compounds of those compounds described in PCT/EP09/060793 (international filing date 20 August 2009) (hereafter the '793 application) and reproduced below. Comparator A, which is described in the '793 application, is 7-cyclopentyl-2-(5-piperazin-1-yl-pyridin-2-ylamino)-7H-pyrrolo[2,3-d]pyrimidine-6-carboxylic acid dimethylamide and is the non-deuterated version of Example 1. Comparator B, which is described in the '793 application, is 7-cyclopentyl-2-(5-piperazin-1-yl-pyridin-2-ylamino)-7H-pyrrolo[2,3-d]pyrimidine-6-carboxylic acid methylamide and is the non-deuterated version of Example 2.

### Pharmacology Studies

Starting with either Example 1 or Comparator A, Sprague Dawley rats were dosed either by intravenous injection (1 mg/kg, n=2) or by oral gavage (5 mg/kg, n=3) and blood samples were collected up to 7 hours. The plasma was separated, frozen and later analyzed. In the case of Example 1 the plasma was analyzed for Example 1 and its mono-methyl metabolite, Example 2. In the case of Comparator A the plasma was analyzed for Comparator A and its mono-methyl metabolite, Comparator B. The quantitation of each metabolite and its parent compound was done by LC-MS/MS with standard curves using authentic standards of each compound. The analysis allowed the characterization of the time-concentration profile and the calculation of pharmacokinetic (PK) parameters via non-compartmental methods. The lowest limit of quantitation was generally around 2 nM for these compounds.

To adequately compare the metabolism of Example 1 and Comparator A, these two compounds were dosed side by side in rats at 1 mg/kg iv and 5 mg/kg po. The observed clearance at 1 mg/kg iv dose for Example 1 was similar to Comparator A, while the VDss of Example 1 is approximately 1.6- fold higher than the VDss of Comparator A, resulting in a comparable half-lives of 3.2 hours and 2.3 hours, respectively. At 5 mg/kg po dose, the absolute oral bioavailability was 1.8-fold higher for Example 1 than Comparator A.

**TABLE 2. PHARMACOKINETIC PROFILES (IV, 1 mg/kg, n=2)**

| Mean PK Parameter | Example 1 | Example 2 (metabolite of 1) | Comparator A | Comparator B (metabolite of A) |
|---|---|---|---|---|
| AUC_{0·7h} (nM*Hours) | 883 | 66 | 1028 | 156 |
| CL (mL/min/kg) | 37 | | 34 | |
| Vdss (L/kg) | 8.6 | | 5.4 | |
| T1/2 (Hours) | 3.2 | | 2.3 | |

**TABLE 3. PHARMACOKINETIC PROFILES (PO, 5 mg/kg, n=3)**

| Mean PK Parameter | Example 1 | Example 2 (metabolite of 1) | Comparator A | Comparator B (metabolite of A) |
|---|---|---|---|---|
| AUC_{0·7h} (nM*Hours) | 2432 | 128 | 1618 | 372 |
| Cmax (nM) | 549 | 25 | 348 | 73 |
| Tmax (Hours) | 2 | 3.3 | 1.5 | 4.3 |
| F (%) | 55 | not determined | 31 | not determined |

From the data analysis in Table 4 the oral plasma exposure AUC0-7h ratio of Example 1 to its metabolite, Example 2, at 5 mg/kg dose is approximately 4-fold lower compared to their non-deuterated versions, Comparators A and B, respectively. This significant difference in the metabolic ratios indicates a slower conversion rate for Example 1 to its metabolite as compared to Comparator A and its metabolite. Since the site of deuteration is also the site of *in vivo* metabolism, the methyl groups of the C6-amide functionality, it can be concluded that replacing the hydrogens with deuteriums on the dimethylamide functionality leads to a reduction in *in vivo* metabolism and an increase in oral exposure of the parent compound.

**TABLE 4. METABOLISM RATIOS (PO, 5 mg/kg, n=3)**

| Mean PK Parameter | Example 1/Example 2 (deuterated compounds) | Comparator A/Comparator B (non-deuterated compounds) |
|---|---|---|
| AUC₀₋₇ₕ (nM*Hours) | 0.05 | 0.23 |
| Cmax (nM) | 0.05 | 0.21 |

## Claims

1. A compound according to formula (I) 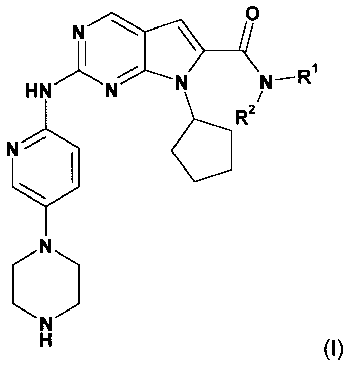 wherein:
R¹ is hydrogen, methyl, CH₂D, CHD₂, or CD₃; and
R² is CH₂D, CHD₂, or CD₃; or a salt thereof.

2. The compound according to claim 1 wherein R¹ is hydrogen, methyl, or CD₃; and R² is CD₃; or a salt thereof.

3. The compound according to claim 1 or 2, wherein the salt is a pharmaceutically acceptable salt.

4. A composition comprising a compound according to claim 1 or claim 2, or a pharmaceutically acceptable salt thereof, and a pharmaceutically acceptable carrier or excipient.

5. A compound according to claim 1 or claim 2, or a pharmaceutically acceptable salt thereof, for use in the treatment of cancer.

6. A compound according to claim 1 or claim 2, or a pharmaceutically acceptable salt thereof, for use in the treatment of a disorder or a disease mediated by CDK4/6, wherein the disorder or the disease is selected from the group consisting of mantle cell lymphoma, multiple myeloma, breast cancer, squamous cell esophageal cancer, liposarcoma, T-cell lymphoma melanoma, non small cell lung cancer, and pancreatic cancer.

7. The compound according to claim 1 which is 7-Cyclopentyl-2-(5-piperzin-1-yl-pyridin-2-ylamino)-7H-pyrrolo[2,3-d]pyrimidine-6-carboxylic acid dimethylamide-d₆ having the following formula 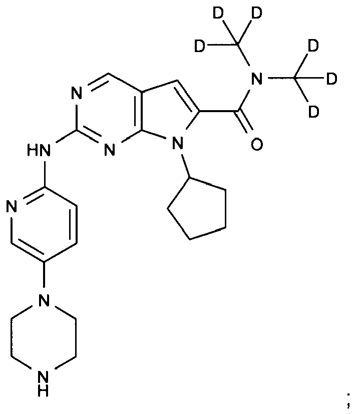 or a salt thereof.

8. The compound according to claim 1 which is 7-Cyclopentyl-2-(5-piperzin-1-yl-pyridin-2-ylamino)-7H-pyrrolo[2,3-d]pyrimidine-6-carboxylic acid methylamide-d₃ having the following formula 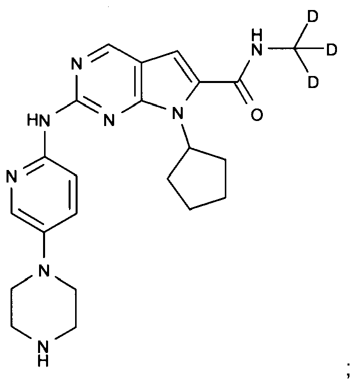 or a salt thereof.

9. The compound according to claim 1 which is 7-Cyclopentyl-2-(5-piperzin-1-yl-pyridin-2-ylamino)-7H-pyrrolo[2,3-d]pyrimidine-6-carboxylic acid methyl(methyl-d₃)amide having the following formula 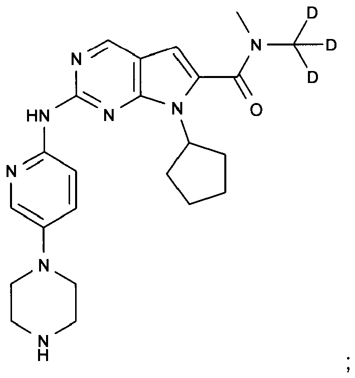 or a salt thereof.

10. The compound according to claim 7, 8, or 9 wherein the salt is a pharmaceutically acceptable salt.

11. A pharmaceutical composition comprising a compound according to claim 7, 8 or 9, or a pharmaceutically acceptable salt thereof, and a pharmaceutically acceptable carrier or excipient.

12. A compound according to claim 7, 8 or 9, or a pharmaceutically acceptable salt thereof, for use in the treatment of cancer.

13. A compound according to claim 7, 8 or 9, or a pharmaceutically acceptable salt thereof, for use in the treatment of a disorder or a disease mediated by CDK4/6, wherein the disorder or the disease is selected from the group consisting of mantle cell lymphoma, multiple myeloma, breast cancer, squamous cell esophageal cancer, liposarcoma, T-cell lymphoma melanoma, non small cell lung cancer, and pancreatic cancer.

## Patentansprüche

1. Verbindung nach Formel (I) 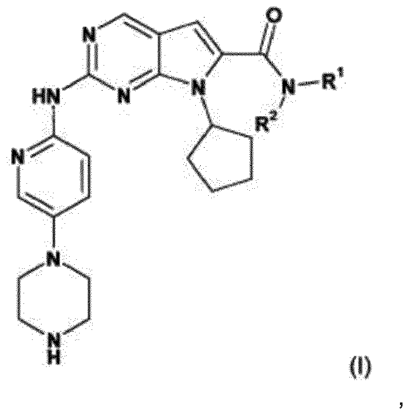 wobei:
R¹ für Wasserstoff, Methyl, CH₂D, CHD₂ oder CD₃ steht; und
R² für CH₂D, CHD₂ oder CD₃ oder ein Salz davon steht.

2. Die Verbindung nach Anspruch 1, wobei R¹ für Wasserstoff, Methyl oder CD₃ steht und R² für CD₃ oder ein Salz davon steht.

3. Die Verbindung nach Anspruch 1 oder 2, wobei das Salz ein pharmazeutisch verträgliches Salz ist.

4. Zusammensetzung, die eine Verbindung nach Anspruch 1 oder 2 oder ein pharmazeutisch verträgliches Salz davon umfasst, und einen pharmazeutisch verträglichen Träger oder Hilfsstoff.

5. Verbindung nach Anspruch 1 oder 2 oder ein pharmazeutisch verträgliches Salz davon zur Verwendung bei der Behandlung von Krebs.

6. Verbindung nach Anspruch 1 oder Anspruch 2 oder ein pharmazeutisch verträgliches Salz davon zur Verwendung bei der Behandlung einer Störung oder einer Erkrankung, die durch CDK4/6 vermittelt wird, wobei die Störung oder die Erkrankung ausgewählt ist aus der Gruppe bestehend aus Mantelzelllymphom, multiplem Myelom, Brustkrebs, Plattenepithelspeiseröhrenkrebs, Liposarkom, T-Zell-Lymphom, Melanom, nicht-kleinzelligem Lungenkrebs und Bauchspeicheldrüsenkrebs.

7. Die Verbindung nach Anspruch 1, welche 7-Cyclopentyl-2-(5-piperzin-1-yl-pyridin-2-ylamino)-7H-pyrrolo[2,3-d]pyrimidin-6-carbonsäuredimethylamid-d₆ mit der folgenden Formel 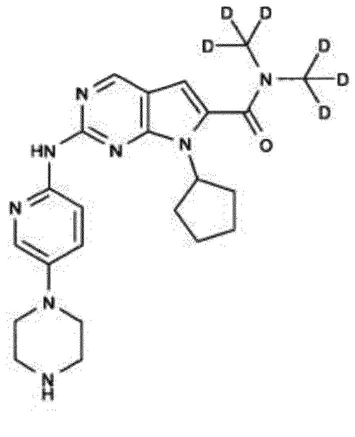 oder ein Salz davon ist.

8. Die Verbindung nach Anspruch 1, welche 7-Cyclopentyl-2-(5-piperzin-1-yl-pyridin-2-ylamino)-7H-pyrrolo[2,3-d]pyrimidin-6-carbansäuremethylamid-d₃ mit der folgenden Formel 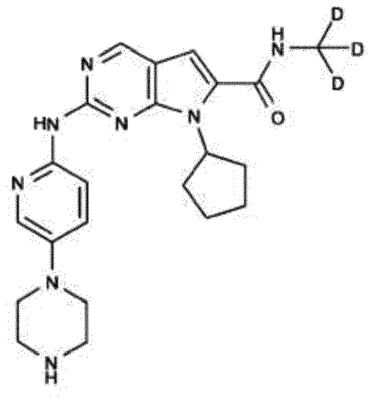 oder ein Salz davon ist.

9. Die Verbindung nach Anspruch 1, welche 7-Cyclopentyl-2-(5-piperzin-1-yl-pyridin-2-ylamino)-7H-pyrrolo[2,3-d]pyrimidin-6-carbonsäuremethyl(methyl-d₃)amid mit der folgenden Formel 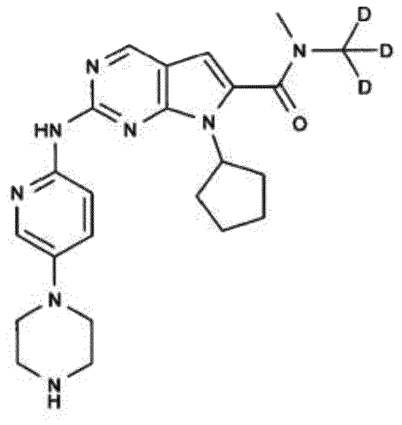 oder ein Salz davon ist.

10. Die Verbindung nach Anspruch 7, 8 oder 9, wobei das Salz ein pharmazeutisch verträgliches Salz ist.

11. Pharmazeutische Zusammensetzung, die eine Verbindung nach Anspruch 7, 8 oder 9 oder ein pharmazeutisch verträgliches Salz davon umfasst, und einen pharmazeutisch verträglichen Träger oder Hilfsstoff.

12. Verbindung nach Anspruch 7, 8 oder 9 oder ein pharmazeutisch verträgliches Salz davon zur Verwendung bei der Behandlung von Krebs.

13. Verbindung nach Anspruch 7, 8 oder 9 oder ein pharmazeutisch verträgliches Salz davon zur Verwendung bei der Behandlung einer Störung oder einer Erkrankung, die durch CDK4/6 vermittelt wird, wobei die Störung oder die Erkrankung ausgewählt ist aus der Gruppe bestehend aus Mantelzelllymphom, multiplem Myelom, Brustkrebs, Plattenepithelspeiseröhrenkrebs, Liposarkom, T-Zell-Lymphom, Melanom, nicht-kleinzelligem Lungenkrebs und Bauchspeicheldrüsenkrebs.

## Revendications

1. Composé selon la formule (I) : 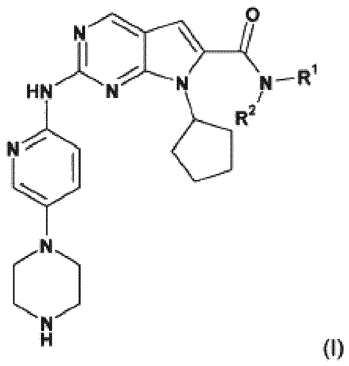 dans laquelle :
- R¹ représente hydrogène, méthyle, CH₂D, CHD₂ ou CD₃ ; et
- R² représente CH₂D, CHD₂ ou CD₃ ;
ou un sel de celui-ci.

2. Composé selon la revendication 1, dans lequel R¹ représente hydrogène, méthyle ou CD₃ ; et R² représente CD₃ ; ou un sel de celui-ci.

3. Composé selon l'une des revendications 1 ou 2, dans lequel le sel est un sel pharmaceutiquement acceptable.

4. Composition comprenant un composé selon la revendication 1 ou la revendication 2, ou un sel pharmaceutiquement acceptable de celui-ci, et un support ou excipient pharmaceutiquement acceptable.

5. Composé selon la revendication 1 ou la revendication 2, ou un sel pharmaceutiquement acceptable de celui-ci, en vue d'une utilisation dans le traitement du cancer.

6. Composé selon la revendication 1 ou la revendication 2, ou un sel pharmaceutiquement acceptable de celui-ci, en vue d'une utilisation dans le traitement d'un trouble ou d'une maladie à médiation par CDK4/6, le trouble ou la maladie étant choisi dans le groupe consistant en le lymphome à cellules du manteau, le myélome multiple, le cancer du sein, le cancer de l'oesophage à cellules squameuses, le liposarcome, le lymphome T, le mélanome, le cancer du poumon non à petites cellules et le cancer du pancréas.

7. Composé selon la revendication 1 qui est le diméthylamide-d₆ de l'acide 7-cyclopentyl-2-(5-pipérazin-1-yl-pyridin-2-ylamino)-7H-pyrrolo[2,3-d]pyrimidine-6-carboxylique ayant la formule suivante : 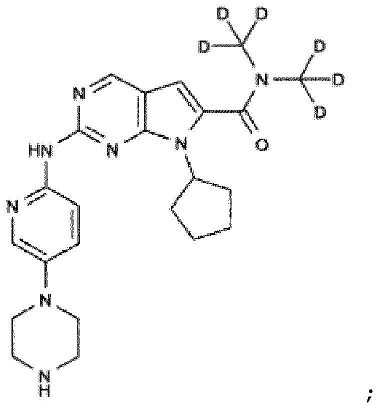 ou un sel de celui-ci.

8. Composé selon la revendication 1 qui est le méthylamide-d₃ de l'acide 7-cyclopentyl-2-(5-pipérazin-1-yl-pyridin-2-ylamino)-7H-pyrrolo[2,3-d]pyrimidine-6-carboxylique ayant la formule suivante : 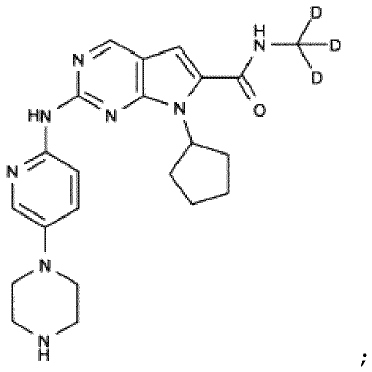 ou un sel de celui-ci.

9. Composé selon la revendication 1, qui est le méthyl(méthyl-d₃)amide de l'acide 7-cyclopentyl-2-(5-pipérazin-1-yl-pyridin-2-ylamino)-7H-pyrrolo[2,3-d]pyrimidine-6-carboxylique ayant la formule suivante : 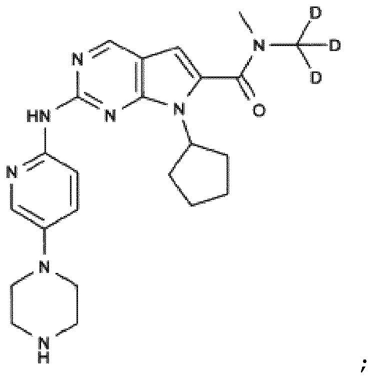 ou un sel de celui-ci.

10. Composé selon l'une des revendications 7, 8 ou 9, dans lequel le sel est un sel pharmaceutiquement acceptable.

11. Composition pharmaceutique comprenant un composé selon l'une des revendications 7, 8 ou 9 ou un sel pharmaceutiquement acceptable de celui-ci et un support ou excipient pharmaceutiquement acceptable.

12. Composé selon l'une des revendications 7, 8 ou 9, ou un sel pharmaceutiquement acceptable de celui-ci, en vue d'une utilisation dans le traitement du cancer.

13. Composé selon l'une des revendications 7, 8 ou 9, ou un sel pharmaceutiquement acceptable de celui-ci, en vue d'une utilisation dans le traitement d'un trouble ou d'une maladie à médiation par CDK4/6, le trouble ou la maladie étant choisi dans le groupe consistant en le lymphome à cellules du manteau, le myélome multiple, le cancer du sein, le cancer de l'oesophage à cellules squameuses, le liposarcome, le lymphome T, le mélanome, le cancer du poumon non à petites cellules et le cancer du pancréas.
